(19) Europäisches Patentamt
European Patent Office
Office européen des brevets

(11) EP 0 596 358 B1

(12) EUROPÄISCHE PATENTSCHRIFT

(45) Veröffentlichungstag und Bekanntmachung des Hinweises auf die Patenterteilung:
**28.10.1998 Patentblatt 1998/44**

(21) Anmeldenummer: **93117239.9**

(22) Anmeldetag: **25.10.1993**

(51) Int Cl.[6]: **C07C 217/84**, C07C 211/52, C07C 211/51, C07C 229/56, C07C 255/58, C07C 217/90, C07C 229/64, C07C 229/60, C07C 323/36, C07C 323/63

(54) **Fluorakyl(en)gruppen enthaltende o-Phenylendiamine und ihre Verwendung als Zwischenverbindungen in der Synthese von Benzimidazolen**

0-Phenylenediamines containing fluoroakyl(ene) groups and their use as intermediates in the preparation of benzimidazoles

O-Phénylène diamines ayant des groupements fluoroalkyles et leur utilisation comme intermédiaire dans la préparation de benzimidazol

(84) Benannte Vertragsstaaten:
**BE CH DE ES FR GB IT LI NL**

(30) Priorität: **06.11.1992 DE 4237564**

(43) Veröffentlichungstag der Anmeldung:
**11.05.1994 Patentblatt 1994/19**

(73) Patentinhaber: **BAYER AG**
**51368 Leverkusen (DE)**

(72) Erfinder:
- **Marhold, Albrecht, Dr.**
  **D-51373 Leverkusen (DE)**
- **Baasner, Bernd, Dr.**
  **D-51467 Bergisch Gladbach (DE)**
- **Lieb, Folker, Dr.**
  **D-51375 Leverkusen (DE)**

(56) Entgegenhaltungen:
**EP-A- 0 251 012**
**EP-A- 0 251 013**
**EP-A- 0 251 014**
**EP-A- 0 487 286**
**DE-A- 1 670 786**
**FR-A- 2 594 437**
**GB-A- 2 163 154**

- **CHEMICAL ABSTRACTS, vol. 118, no. 21, 24. Mai 1993, Columbus, Ohio, US; abstract no. 213074u, M. ENOMOTO ET AL. 'Preparation of 2-cyano-1-dimethylsulfamoyl-5,6-bis(1',1',2',2'-te trafluoroethoxy)benzimidazole as horticultural and agrochemical fungicide.' Seite 919 ; & JP-A-04 308 580 (SUMITOMO CHEMICAL CO.) 30. Oktober 1992**
- **CHEMICAL ABSTRACTS, vol. 60, no. 2, 20. Januar 1964, Columbus, Ohio, US; abstract no. 1732f, L. M. YAGUPOL'SKII ET AL. 'Synthesis of 5-methyl-6-trifluoromethylbenzimidazole' & ZH. OBSHCH. KHIM. Bd. 33, Nr. 9 , 1963 Seiten 3031 - 3035**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 596 358 B1

Printed by Jouve, 75001 PARIS (FR)

## Beschreibung

In der EP-A 2-251 013 und der EP-A 1-487 286 sind andere o-Phenylendiamine beschrieben als diejenigen, die Gegenstand der vorliegenden Erfindung sind.

Aus der JP-A 1 135 773 sind o-Phenylendiamine bekannt, die am aromatischen Kern ausschließlich mit Halogen substituiert sind sowie daraus herstellbare Benzimidazole mit insektizider und akarizider Wirkung, die am Stickstoffatom in 1-Stellung unsubstituiert sind und in 2-Stellung einen mindestens 3 C-Atome enthaltenden Substituenten aufweisen.

Aus der US-A 3 755 346 sind Benzimidazole mit insektizider, herbizider und nematozider Wirkung bekannt, die am aromatischen Kern einen Nitro-Substituenten enthalten und am Stickstoffatom in 1-Stellung unsubstituiert sind. Solche Benzimidazole können aus geeignet substituierten o-Phenylendiaminen hergestellt werden, also aus solchen, die am aromatischen Kern einen Nitro-Substituenten enthalten.

Die vorliegende Erfindung betrifft Fluoralkyl(en)gruppen enthaltende o-Phenylendiamine der Formel (I)

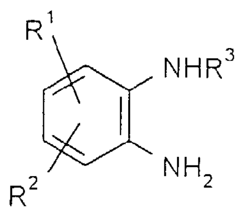

(I)

in der

$R^1$ für $CF_3$, $OCF_3$, $SCF_3$, $SO_2$-$C_1$-$C_6$-Alkyl; das geradkettig oder verzweigt und durch Fluor ganz oder teilweise substituiert sein kann, $N(CF_3)_2$, einen Phenyl- oder Phenoxyrest mit $CF_3$ oder CN in 4-Position und gegebenenfalls weiteren Substituenten, 1,1,2,3,3,3-Hexafluorpropoxy, 1,1,2-Trifluor-2-chlor-ethoxy, 1,1,2,2-Tetrafluorethoxy, 1,1,2-Trifluor-2-chlor-ethylthio oder 1,1,2,3,3,3-Hexafluorpropylthio, unabhängig davon

$R^2$ für F, Cl, Br, CN, $CH_3$, $OCF_3$, $SO_2$-$C_1$-$C_6$-Alkyl, das geradkettig oder verzweigt und durch Fluor ganz oder teilweise substituiert sein kann, COO-$C_1$-$C_6$-Alkyl, $COOC_6H_5$, 1,1,2,2-Tetrafluorethoxy, 1,1,2,3,3,3-Hexafluorpropoxy oder 1,1,2-Trifluor-2-chlor-ethoxy und

$R^3$ für Wasserstoff, $COCH_3$ oder $COCF_3$ stehen, wobei weiterhin

$R^1$ und $R^2$ gemeinsam für einen -O-CFCl-CFCl-O-Rest stehen können,

mit Ausnahme von Verbindungen, bei denen

$R^1$ für 1,1,2,2-Tetrafluorethoxy und $R^2$ für Cl oder Brom oder

$R^1$ für $CF_3$ oder 1,1,2,2-Tetrafluorethoxy und $R^2$ für $CF_3$, $OCF_3$, 1,1,2,2-Trifluorethoxy oder 1,1,2-Trifluor-2-chlorethoxy stehen und wobei mindestens einer der Reste $R^1$, $R^2$ und $R^3$ im Rahmen der oben gegebenen Definitionen für eine Fluoralkyl(en) enthaltende Gruppe steht.

Bevorzugte Fluoralkylgruppen enthaltende o-Phenylendiamine der Formel (I) enthalten folgende Reste $R^1$ und $R^2$ in der jeweils angegebenen Position, wobei $R^3$ stets Wasserstoff bedeutet:

| $R^1$ | $R^2$ | $R^1$ | $R^2$ |
|---|---|---|---|
| 4-$CF_3O$ | 5-Br | H | 4-$N(CF_3)_2$ |
| 4-$CF_3O$ | 5-Cl | H | 4-$CF_3SO_2$ |
| 4-$CF_3S$ | 5-Cl | 4-$CF_3$ | 6-$COOCH_3$ |

(fortgesetzt)

| $R^1$ | $R^2$ | $R^1$ | $R^2$ |
|---|---|---|---|
| | | $CF_3$ | 6-$COOC_2H_5$ |
| 3-$CF_3$ | 5-$CH_3$ | 4-$CF_3$ | 6-$COOiC_3H_7$ |
| 3-$CF_3$ | 5-Cl | 4-$CF_3$ | 6-$COOC_6H_5$ |
| 3-$CF_3$ | 5-Br | 4-$CF_3$ | 5-$COOCH_3$ |
| 3-$CF_3$ | 5-CN | 4-$CF_3$ | 5-$COOC_2H_5$ |
| 5-$CF_3S$ | 3-Br | 4-$CF_3$ | 5-$COOtC_4H_9$ |
| 5-$CF_3$ | 3-CN | 5-$CF_3S$ | 3-Br |
| 3-$CF_3$ | 4-Cl | 5-$CF_3S$ | 3-Cl |
| 3-$CF_3O$ | 4-Cl | 5-$CF_3O$ | 3-$COOCH_3$ |
| 3-$CF_3$ | 4-CN | 5-$CF_3O$ | 3-$COOC_2H_5$ |
| 5-(2,6-dichlor-4-trifluormethyl-phenoxy) | 3-Cl | 5-$CF_3O$ | 3-$COOiC_3H_7$ |
| | | 5-$CFClHCF_2S$ | 3-$COOCH_3$ |
| 5-(2,6-dichlor-4-trifluormethyl-phenoxy) | 4-Cl | 5-$CFClHCF_2S$ | 3-$COOC_2H_5$ |
| | | 5-$CFClHCF_2S$ | 3-$COOiC_3H_7$ |
| | | 5-$CFClHCF_2S$ | 3-Br |
| | | 5-Hexafluorpropylthio | 3-Br |
| 4-Hexafluorpropoxy | 5-Cl | 3-$CF_3$ | 5-$COOC_4H_9$ |
| | | 5-$CF_3O$ | 3-$COOCH_3$ |
| 5-$CF_3O$ | 3-$COOC_2H_5$ | 5-$CF_3O$ | 3-$COOC_4H_9$ |
| | | 4-$CF_3CHFCF_2O$ | 5-$CF_3CHFCF_2O$ |
| 4-$CFClHCF_2O$ | 5-$CFClHCF_2O$ | 5-$CFClHCF_2O$ | 4-Cl |
| 4-$CF_3$ | 5-$CF_3$-CHF-CFO | | |
| | | 5-$CF_3SO_2$ | 3-Br |
| | | 5-$CF_3SO$ | 3-Cl |

Weitere bevorzugte Verbindungen der Formel (I) entsprechen der Formel (I')

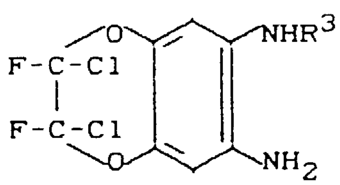

(I'),

in der

$R^3$ die bei Formel (I) angegebene Bedeutung hat.

Die Verbindungen der Formel (I) können, abhängig von der Art der Reste $R^1$ und $R^2$ auf verschiedene Weise hergestellt werden.

Sollen Verbindungen der Formel (I) hergestellt werden, bei denen sowohl $R^1$ als auch $R^2$ eine Donorgruppe in 4-und 5-Stellung zu den Aminogruppen darstellen, z.B. Verbindungen, bei denen $R^1$ für Polyfluoralkoxy oder Poly-

fluoralkylthio und $R^1$ und/oder $R^2$ für Fluor, Chlor, Brom, Alkyl, Alkoxy oder Bisfluoralkylamino steht, so kann man ein Benzolderivat der Formel (II)

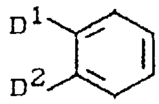

(II),

in der

$D^1$ für $CF_3O$, $CF_3S$, $CHF_2CF_2O$, $CHFCl\text{-}CF_2O$, $CF_3CHFCF_2O$, $CF_3CF_2O$, $CF_3CF_2CF_2O$, $CF_3CF_2S$ oder $CF_3CHFCF_2O$ und

$D^2$ für $CF_3O$, $CF_3S$, $CHF_2CF_2O$, $CHFCl\text{-}CF_2O$, $CF_3CHF\text{-}CF_2O$, $CF_3CF_2O$, $CF_3CF_2CF_2O$, $CF_3CF_2S$, $CF_3CHFCF_2O$, Fluor, Chlor, Brom, $C_1$-$C_6$-Alkyl oder $C_1$-$C_6$-Alkoxy

steht

dinitrieren, die Nitrogruppen anschließend reduzieren und so Verbindungen der Formel (I) erhalten, bei denen $R^1$ und $R^2$ in 4- und 5-Stellung zu den Aminogruppen stehen und die Bedeutung von $D^1$ und $D^2$ (siehe Formel (II)) haben. Die Dinitrierung kann z.B. mit $HND_3/H_2SO_4$-Gemischen, die gegebenenfalls auch Oleum enthalten können, und bei Temperaturen von z.B. 0 bis 100°C durchgeführt werden. Die Reduktion kann beispielsweise mit Eisen in Gegenwart von wäßriger Salzsäure und Ethanol bei Temperaturen von z.B. 50 bis 100°C oder katalytisch mit elementarem Wasserstoff bei z.B. 25 bis 100°C, z.B. 1 bis 100 bar und in Gegenwart von Metallen oder Metallverbindungen der 8.Nebengruppe des periodischen Systems, insbesondere Nickel oder Palladium, enthaltenden Katalysatoren durchgeführt werden.

Sollen Verbindungen der Formel (I) hergestellt werden, bei denen $R^1$ die bei Formel (I) angegebene Bedeutung hat und in 4-Stellung zu den Aminogruppen steht und $R^2$ für C1 oder Br in 5-Stellung zu den Aminogruppen steht, so kann man z.B. ein Nitrobenzolderivat der Formel (III)

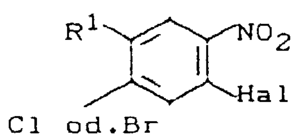

(III),

in der

$R^1$ die bei Formel (I) angegebene Bedeutung hat und Hal für Fluor, Chlor oder Brom steht,

mit Ammoniak umsetzen, so die Hal-Gruppe gegen eine Aminogruppe austauschen und das so erhaltene Nitranilin reduzieren. Der Austausch von Halogen gegen eine Aminogruppe kann z.B. mit flüssigem Ammoniak in Gegenwart von Wasser und einem Tetraalkylammoniumsalz bei Temperaturen von z.B. 80 bis 200°C in einem Druckgefäß durchgeführt werden. Die Reduktion des Nitranilins kann z.B. analog zu der oben beschriebenen Reduktion von Dinitroverbindungen erfolgen.

Sollen Verbindungen der Formel (I) hergestellt werden, bei denen $R^1$ die bei Formel (I) angegebene Bedeutung hat und in 4-Stellung zu den Aminogruppen steht, $R^2$ für Chlor oder Brom in 6-Stellung zu den Aminogruppen steht und $R^3$ Wasserstoff bedeutet, so kann man z.B. ein Nitranilin der Formel (IV)

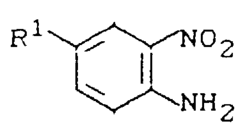

(IV),

in der

$R^1$ die bei Formel (I) angegebene Bedeutung hat

mit einem Chlorierungs- oder Bromierungsmittel umsetzen, so ein Chlor- oder Bromatom in die meta-Stellung zur Nitrogruppe einführen und anschließend die Nitrogruppe reduzieren. Als Chlorierungs- bzw, Bromierungsmittel kommen elementares Chlor, elementares Brom und andere übliche Chlorierungs- und Bromierungsmittel in Frage. Geeignete Lösungsmittel sind beispielsweise Wasser, verdünnte Mineralsäuren, Essigsäure, Chloralkane und Trifluoressigsäure und geeignete Temperaturen beispielsweise solche von -20 bis +50°C. Die Reduktion kann z.B. analog zu der oben beschriebenen Reduktion von Dinitroverbindungen erfolgen.

Sollen Verbindungen der Formel (I) hergestellt werden, bei denen $R^1$ eine Donorgruppe in 4-Stellung zu den beiden Aminogruppen, $R^2$ eine Akzeptorgruppe, z.B. COO-$C_1$-$C_6$-Alkyl, CN, $CF_3$ oder $SO_2$-$C_1$-$C_6$-Alkyl darstellt und $R_3$ ungleich Wasserstoff ist, so kann man z.B. ein Benzolderivat der Formel (V)

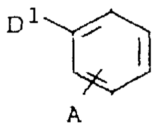

(V),

in der

$D^1$ die bei Formel (II) angegebene Bedeutung hat und

A für $CF_3$, $SO_2$-$C_1$-$C_6$-Alkyl, das geradkettig oder verzweigt und durch Fluor ganz oder teilweise substituiert sein kann, COO-$C_1$-$C_6$-Alkyl oder CN steht,

mononitrieren (Eintritt der $NO_2$-Gruppe in para-Position zu $D^1$), die $NO_2$-Gruppe zur $NH_2$-Gruppe reduzieren, die $NH_2$-Gruppe z.B. mit Essigsäure oder Trifluoressigsäure acylieren, nochmals mononitrieren (Eintritt dieser $NO_2$-Gruppe in ortho-Position zur NHCOR-Gruppen mit R = z.B. $CH_3$ oder $CF_3$), diese $NO_2$-Gruppe zur $NH_2$-Gruppe reduzieren und gegebenenfalls, wenn man eine Verbindung der Formel (I) mit $R^3$ = Wasserstoff herstellen will, die Acylgruppe durch Verseifung abspalten.

Die Nitrierungen kann man z.B. mit Salpetersäure in einem geeigneten organischen Lösungsmittel bei Temperaturen zwischen z.B. 0 und 50°C durchführen, die Reduktionen z.B. analog zu den oben beschriebenen Reduktionen von Dinitroverbindungen und die Einführung und gegebenenfalls durchzuführende Abspaltung der Acyl-Gruppe nach üblichen Methoden zum Schützen von Aminogruppen.

Bei dem Verfahren, bei dem eine Dinitrierung und eine Reduktion vorgenommen wird ist überraschend, daß die beiden Nitrogruppen mit hoher Selektivität ortho-ständig zueinander eingeführt werden können und trotz relativ drastischer Reaktionsbedingungen bei der Dinitrierung Fluoralkoxy- und Fluoralkylthiogruppen nicht verseift oder abgespalten werden,

Bei dem Verfahren, bei dem ein Halogen gegen eine $NH_2$-Gruppe durch Verdrücken mit Ammoniak ausgetauscht wird ist überraschend, daß Selektiv ein Halogen und dieses Selektiv in ortho-Postion zur $NO_2$-Gruppe ausgetauscht werden kann.

Die erfindungsgemäßen Fluoralkyl(en)gruppen enthaltenden o-Phenylendiamine, in denen $R^3$ $COCH_3$ oder $COCF_3$ bedeutet, können durch Hydrolyse in o-Phenylendiamine der Formel (I) mit $R^3$ = Wasserstoff überführt werden. Aus o-Phenylen-diaminen der Formel (I) mit $R^3$ = Wasserstoff oder $COCF_3$ können substituierte Benzimidazole der Formel

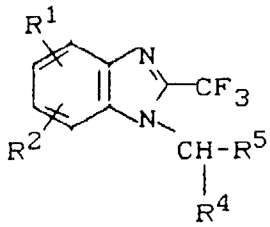

(VI)

erhalten werden, indem man Stoffe der Formel (I) mit $R^3$ = H oder $COCF_3$ zunächst mit Trifluoressigsäure zu 2-Trifluormethylbenzimidazolen der Formel

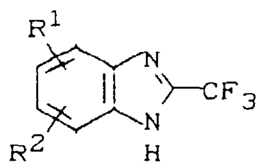

(VII)

umsetzt und dann eine weitere Umsetzung mit Verbindungen der Formel

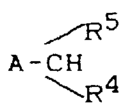

(VIII)

vornimmt, wobei in den Formeln (VI), (VII) und (VIII)

$R^1$ und $R^2$ den obigen Bedeutungsumfang annehmen,

$R^4$ für Wasserstoff, Alkyl, Alkoxy oder für gegebenenfalls substituiertes Aryl steht,

$R^5$ für Hydroxy, Cyano oder für jeweils gegebenenfalls substituiertes Alkyl, Alkenyl, Alkinyl, Alkoxy, Alkenyloxy, Alkinyloxy, Alkylthio, Amino, Aminocarbonyl, Alkylcarbonyl, Alkoxycarbonyl, Alkylcarbonyloxy, Dialkoxyphosphonyl, (Hetero)Aryl, (Hetero)Arylcarbonyl, (Hetero)Aryloxycarbonyl, (Hetero)Arylcarbonyloxy oder (Hetero)Arylaminocarbonylaminocarbonyloxy steht und

A eine geeignete Abgangsgruppe bedeutet.

Abgangsgruppen sind dem Fachmann bekannt und sind beispielsweise Halogen, Alkyl-, Alkoxy- oder Arylsulfonyloxy, Hydroxy oder Alkoxy.

Substituierte Benzimidazole der Formel (VI) sind Wirkstoffe zur Bekämpfung von tierischen Schädlingen, Arthropoden und Nematoden, insbesondere Insekten und Spinnentieren, die in der Landwirtschaft, in Forsten, im Vorrats- und Materialschutz sowie auf dem Hygienesektor vorkommen.

Beispiele

Beispiele 1 bis 6 (Dinitrierung und Reduktion)

Beispiel 1

Zu 500 g einer Mischsäure enthaltend 33 Gew.-% $HNO_3$ und 67 Gew.-% $H_2SO_4$ wurden 320 g 1,2-Bis-(2-chlor-1,1,2-trifluorethoxy)-benzol getropft. Nach einer Stunde bei 40°C wurden 250 ml 20 gew.-%iges Oleum zugetropft. Anschließend wurde auf 80°C erhitzt und 15 Stunden lang nachgerührt, Dann wurden weitere 120 ml 20 gew.-%iges Oleum und 250 g der oben angegebenen Mischsäure zugetropft. Nach 6 Stunden bei 80 bis 82°C wurde abgekühlt

und auf Eis gegossen, Die organische Phase wurde abgetrennt und mit Wasser gewaschen. Nach azeotroper Trocknung mit 1,2-Dichlorethan wurden 350 g 98 Gew.-% reines 1,2-Dinitro-4,5-bis-(2-chlor-1,1,2-trifluorethoxy)-benzol erhalten (Öl, $n_D^{20}$ : 1,4832, GC 99,1%),

350 g dieser Dinitroverbindung wurden zu einem Gemisch aus 1,5 l Ethanol, 50 ml Wasser, 30 ml konzentrierter wäßriger Salzsäure und 470 g Eisenspänen getropft und insgesamt 15 Stunden zum Sieden am Rückfluß erhitzt. Danach wurde die erkaltete Lösung abfiltriert, eingeengt und der Rückstand aus Cyclohexan umkristallisiert. Es wurden 216 g 1,2-Diamino-4,5-bis-(2-chlor-1,1,2-trifluorethoxy)-benzol mit einem Schmelzpunkt von 58 bis 60°C erhalten.

Beispiel 2

Analog Beispiel 1 wurde aus 1,2-Bis-(1,1,2,3,3,3-hexafluorpropoxy)-benzol die entsprechende 4,5-Dinitroverbindung (Öl, $n_D^{20}$ : 1,4852) und die entsprechende 4,5-Diaminoverbindung (Öl, 87 Gew.-% rein) hergestellt.

Beispiel 3

Analog Beispiel 1 wurde aus 1-(1,1,2-Trifluor-2-chlorethoxy)-2-chlorbenzol die entsprechende 4,5-Dinitroverbindung (Schmelzpunkt 56 bis 57°C) und die entsprechende 4,5-Diaminoverbindung (Schmelzpunkt 67 bis 68°C) hergestellt.

Beispiel 4

Analog Beispiel 1 wurde aus 1-Trifluormethoxy-2-brombenzol die entsprechende 4,5-Dinitroverbindung (Schmelzpunkt 73 bis 75°C) und die entsprechende 4,5-Diaminoverbindung (Öl, 98 Gew.-% rein, $n_D^{20}$ : 1,5485) hergestellt.

Beispiel 5

Analog Beispiel 1 wurde aus 1-Trifluormethoxy-2-chlorbenzol die entsprechende 4,5-Dinitroverbindung (Schmelzpunkt 55 bis 56°C) und die entsprechende 4,5-Diaminoverbindung (Schmelzpunkt 56-57°C) hergestellt.

Beispiel 6

Aus 1-(1,1,2,3,3,3-Hexafluorpropoxy)-2-chlor-benzol wurde die entsprechende 4,5-Dinitroverbindung (Öl) und die entsprechende 4,5-Diaminoverbindung (öl) hergestellt.

Beispiele 7 bis 12

Verdrückung mit Ammoniak und Reduktion

Beispiel 7

In einem Autoklaven wurden 260 g 3-Nitro-2,5-dichlorbenzotrifluorid, 130 ml Wasser und 10 g Tetraethylammoniumchlorid vorgelegt und 120 ml flüssiges Ammoniak aufgedrückt. Anschließend wurde auf 130°C erhitzt und für 10 Stunden bei dieser Temperatur gerührt. Nach dem Abkühlen wurde der Ansatz abfiltriert, der abgetrennte Niederschlag mit Wasser gewaschen und getrocknet. Es fielen 194 g 2-Amino-3-nitro-5-chlor-benzotrifluorid mit einem Schmelzpunkt von 67°C an.

134 g des wie oben beschrieben erhaltenen Nitranilins wurden in 800 ml Ethanol gelöst, dann 20 ml Wasser, 10 ml konzentrierte wäßrige Salzsäure und 160 g Eisenspäne zugegeben. Die Mischung wurde für 15 Stunden zum Sieden am Rückfluß erhitzt, dann abgekühlt, abgesaugt, der Filterrückstand mit Dichlormethan gewaschen und anschließend die organischen Phasen unter reduziertem Druck vom Lösungsmittel befreit. Es fielen 171 g 5-Chlor-3-trifluormethyl-1,2-diaminobenzol mit einem Schmelzpunkt von 53°C an.

Beispiel 8

Analog Beispiel 7 wurde aus 3-Nitro-4,6-dichlor-difluorchlormethoxybenzol zunächst 3-Nitro-4-amino-6-chlor-difluorchlormethoxybenzol (Schmelzpunkt 73°C) und daraus 3,4-Diamino-6-chlor-difluorchlormethoxybenzol (Öl) erhalten.

Beispiel 9

Analog Beispiel 7 wurde aus 3-Brom-5-nitro-6-chlorbenzotrifluorid zunächst 3-Brom-5-nitro-6-amino-benzotrifluorid (Schmelzpunkt 80 bis 82°C) und daraus 3-Brom-5,6-diamino-benzotrifluorid (Schmelzpunkt 52 bis 54°C) hergestellt.

Beispiel 10

Analog Beispiel 7 wurde aus 3-Cyano-4-chlor-5-nitrobenzotrifluorid zunächst 3-Cyan-4-amino-5-nitro-benzotrifluorid (Schmelzpunkt 99 bis 100°C) und daraus 3-Cyano-4,5-diamino-benzotrifluorid hergestellt.

Beispiel 11

Analog Beispiel 7 wurde aus 3,6-Dichlor-5-nitro-benzotrifluorid zunächst 3-Chlor-5-nitro-6-amino-benzotrifluorid (Schmelzpunkt 53 bis 54°C) und daraus 3-Chlor-5,6-diamino-benzotrifluorid hergestellt.

Beispiel 12

Aus 2-Brom-4-fluor-5-nitro- (1,1,2-trifluor-2-chlor)--ethoxybenzol wurde zunächst 2-Brom-4-amino-5-nitro-(1,1,2-trifluor-2-chlor-ethoxy)-benzol (Schmelzpunkt 90°C) und daraus 2-Brom-4,5-diamino-(1,1,2-trifluor-2-chlor)-ethoxybenzol hergestellt.

Beispiel 13

(Halogenierung eines Nitranilins und Reduktion)

24 g fein gepulvertes 2-Nitro-4-trifluormethylmercaptoanilin wurden in 50 ml Trifluoressigsäure gelöst und bei 20°C 18 g Brom zudosiert. Dann wurde für 3 Stunden bei 20°C und für weitere 30 Minuten bei 40°C nachgerührt, die Mischung auf Wasser gegeben und das Produkt in Dichlormethan aufgenommen. Es fielen nach Entfernung des Lösungsmittels 31 g 6-Brom-2-nitro-4-trifluormethylmercapto-anilin an,

155 g des so hergestellten Nitranilins wurden in 700 ml Ethanol mit 15 ml Wasser, 10 ml konzentrierter wäßriger Salzsäure und 70 g Eisenspänen für 15 Stunden zum Sieden am Rückfluß erhitzt, dann das Gemisch abfiltriert, das Filtrat unter reduziertem Druck vom Lösungsmittel befreit und das feste Rohprodukt aus Cyclohexan umkristallisiert. Es wurden 112 g 6-Brom-4-trifluormethylmercapto-1,2-diaminobenzol mit einem Schmelzpunkt von 60 bis 61°C erhalten.

Beispiel 14

Analog Beispiel 13 wurden 27 g 2-Nitro-4-trifluormethylsulfonylanilin in 100 ml Essigsäure mit 18 g Brom bromiert.

Nach Aufarbeitung fielen 32 g 2-Nitro-6-brom-4-trifluormethylsulfonyl-anilin an, Schmelzpunkt 147°C.

32 g des so hergestellten Nitramins wurde mit Eisenspänen in Alkohol und wäßriger Chlorwasserstoffsäure reduziert, Es fielen 24 g 3-Brom-5-trifluormethylsulfonyl-phenylen-1,2-diamin an, Schmelzpunkt 155-157°C.

Beispiel 15

Analog Beispiel 14 wurden 27 g 2-Nitro-4-trifluormethylsulfonyl-anilin in 100 ml Essigsäure mit 10 g Chlor chloriert. Es fielen 29 g 2-Nitro-4-trifluormethylsulfonyl-6-chlor-anilin an, Schmelzpunkt: 138-139°C.

Durch Reduktion wurden 13 g 3-Chlor-5-trifluormethylsulfonyl-1,2-phenylendiamin (Schmelzpunkt: 143-145°C) erhalten.

Beispiele 16 bis 20

(Nitrierung und Reduktion in 2 Stufen)

Beispiel 16

263 g 4-(2,6-Dichlor-4-trifluormethyl-phenoxy)-acetanilid wurden in 1100 ml Dichlormethan gelöst und bei 10°C vorgelegt. Dann wurden bei dieser Temperatur 88 g 98 gew.-%ige Salpetersäure zugetropft. Es wurde 1 Stunde bei 10°C und 2 weitere Stunden bei 30°C nachgerührt. Nach der Zugabe von 300 ml Wasser wurden die Phasen getrennt

und die organische Phase unter reduziertem Druck vom Dichlormethan befreit. Es verblieben 253 g 2-Nitro-4-(2,6-dichlor-4-trifluormethyl-phenoxy)-acetanilid mit einem Schmelzpunkt von 138 bis 140°C.

91 g des so hergestellten Acetanilids wurden in 800 ml Dioxan gelöst, 10 g Raney-Nickel zugegeben und bei 25 bis 45°C in einer Hydrierapparatur mit maximal 50 bar Wasserstoffdruck hydriert. Nach Entspannen und Filtration wurde das Dioxan bei leichtem Vakuum abdestilliert. Es verblieben 65 g 2-Amino-4-(2,6-dichlor-4-trifluormethyl-phenoxy)-acetanilid mit einem Schmelzpunkt von 222 bis 223°C.

Beispiel 17

Analog zu Beispiel 16 wurde aus 3-Trifluormethyl-4-methoxy-acetanilid zunächst 3-Trifluormethyl-4-methoxy-6-nitro-acetanilid (Schmelzpunkt 143 bis 144°C) und daraus 3-Trifluormethyl-4-methoxy-6-amino-acetanilid (Schmelzpunkt 164 bis 165°C) hergestellt.

Beispiel 18

Analog Beispiel 16 wurde aus 3-Trifluormethyl-4-fluortrifluoracetanilid zunächst 3-Trifluormethyl-4-fluor-6-nitro-trifluoracetanilid (Schmelzpunkt 78°C) und daraus 3-Trifluormethyl-4-fluor-6-amino-trifluoracetanilid (Schmelzpunkt 92 bis 93°C) hergestellt.

Beispiel 19

Analog Beispiel 16 wurde aus 3-Trifluormethyl-4-bromtrifluoracetanilid zunächst 3-Trifluormethyl-4-brom-6-nitro-trifluoracetanilid (Schmelzpunkt 110 bis 112°C) und daraus 3-Trifluormethyl-4-brom-6-amino-trifluoracetanilid (Schmelzpunkt 63 bis 65°C) hergestellt.

Beispiel 20

Analog Beispiel 16 wurde aus 3-Trifluormethylthio-4-chlor-trifluoracetanilid zunächst 3-Trifluormethylthio-4-chlor-6-nitro-trifluoracetanilid (Schmelzpunkt 99-100°C) und daraus 3-Trifluormethylthio-4-chlor-6-aminotrifluoracetanilid (Schmelzpunkt: 88-90°C) hergestellt.

Beispiel 21

0,2 Mol 3-Brom-5-trifluormethyl-phenylen-1,2-diamin wurden mit 150 ml Trifluoressigsäure für 3 Stunden auf Rückflußtemperatur erhitzt. Zur Aufarbeitung wurde überschüssige Trifluoressigsäure abdestilliert und der Rückstand zwischen 100 ml Wasser und 300 ml Essigester verteilt. Die organische Phase wurde abgetrennt, nacheinander mit jeweils 100 ml wäßriger Natriumhydrogencarbonatlösung und Wasser gewaschen, über Natriumsulfat getrocknet und im Vakuum eingeengt. Der Rückstand wurde durch Chromatographie an Kieselgel (Laufmittel: Cyclohexan/Essigester 1:1) gereinigt.

Man erhielt 4-Brom-6-trifluormethyl-2-trifluormethyl-1H-benzimidazol vom Schmelzpunkt 149-151°C.

Beispiel 22

0,03 Mol 4-Brom-6-trifluormethyl-2-trifluormethyl-1H-benzimidazol und 0,06 Mol pulverisiertes Kaliumcarbonat wurden in 70 ml Essigester für 15 Minuten auf Rückflußtemperatur erhitzt, anschließend mit 3,9 g (0,04 Mol) Chlormethyl-methylthioether in 20 ml Essigester versetzt und unter Rühren für weitere 4 Stunden auf Rückflußtemperatur erhitzt. Zur Aufarbeitung wurde die abgekühlte Reaktionsmischung zweimal mit jeweils 40 ml Wasser gewaschen, über Natriumsulfat getrocknet, im Vakuum eingeengt und der Rückstand durch Chromatographie an Kieselgel (Laufmittel: Dichlormethan) gereinigt.

Man erhielt 1-Methylthiomethyl-4-brom-6-trifluormethyl-2-trifluormethyl-benzimidazol vom Schmelzpunkt 56-60°C.

Beispiel 23

a) Bei 20°C wurden zu 157 g 2-(2H-Hexafluorpropoxy)-isopropylbenzol 125 g Mischsäure (33 % Salpetersäure, 67 % Schwefelsäure) getropft. Nach 2 stündigem Rühren bei 20 bis 25°C wurde der Ansatz auf Eis gegossen und anschließend die organische Phase mit Methyl-tert.-butylether extrahiert, Eine Feindestillation der extrahierten organischen Phase lieferte 89 g 2-(2H-Hexafluorpropoxy)-5-nitroisopropylbenzol (Siedepunkt: 142-145°C/20

mbar; $n_D^{20}$ : 1,4562).

b) Eine Mischung aus 89 g 2-(2H-Hexafluorpropoxy)-5-nitro-isopropylbenzol, 5 ml 35-%ige wäßrige Salzsäure, 3 ml Wasser, 80 g Eisenpulver und 420 ml Ethanol wurden 15 Stunden auf Rückfluß erhitzt, dann mit 4 g Natriumhydroxyd versetzt und heiß filtriert. Der Filtrationsrückstand wurde mit Ethanol gewaschen und anschließend das Filtrat gemeinsam mit dem ethanolischen Extrakt destilliert. Es wurden 53 g 3-Isopropyl-4-(2H-hexafluorpropoxy)-anilin erhalten (Siedepunkt: 110-115°C/12 mbar).

c) 30 g 3-Isopropyl-4-(2H-hexafluorpropoxy)-anilin wurden in 150 ml Toluol vorgelegt und eine Mischung aus 40 g Trifluoressigsäureanhydrid und 50 ml Trifluoressigsäure zugetropft. Anschließend wurde für 6 Stunden am Rückfluß gekocht, dann abgekühlt und bei 15 mbar die leicht flüchtigen Anteile abdestilliert. Der verbleibende Rückstand wurde im Hochvakuum destilliert. Ausbeute: 28 g 3-Isopropropyl-4-(2H-hexafluorpropoxy)-N-trifluoracetanilid (Siedepunkt: 110-120°C/0,15 mbar).

d) 18 g 3-Isopropyl-4-(2H-hexafluorpropoxy)-trifluoracetanilid wurden in 50 ml Dichlormethan vorgelegt und bei 20°C 26 g Mischsäure (33 % Salpetersäure, 67 % Schwefelsäure) zugetropft. Nach 5 Stunden Rühren bei 20-25°C wurden 60 g Eis zugegeben, die Phasen getrennt und die organische Phase eingeengt. Es verblieben 19 g Feststoff, der ohne weitere Reinigung umgesetzt wurde.

e) 19 g des gemäß d) erhaltenen 2-Nitro-3-isopropyl-4-(2H-hexafluorpropoxy)-trifluoracetanilids wurden zusammen mit 7 g Eisenpulver, 7 g Eisenspänen, 1,5 ml Salzsäure und 70 ml Ethanol für 20 Stunden auf Rückfluß erhitzt. Anschließend wurde heiß filtriert, der Filterrückstand mit Ethanol nachgewaschen und dann das Filtrat und die ethanolische Lösung vereinigt und vom Lösungsmittel befreit. Der dabei anfallende Rückstand wurde nach dem Erkalten mit n-Hexan überschichtet und angerieben. Nach dem Stehen über Nacht wurde die Flüssigkeit abdekantiert und das verbleibende Festprodukt analysiert. Es handelte sich um 11 g, nach gaschromatografischer Analyse 95,5 % reinem 2-Amino-3-isopropyl-4-(2H-hexafluorpropoxy)-trifluoracetanilid mit einem Schmelzpunkt von 167-168°C.

## Patentansprüche

**1.** Fluoralkyl(en)gruppen enthaltende o-Phenylendiamine der Formel (1)

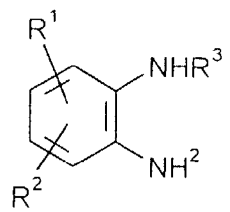

(I)

in der

$R^1$ für $CF_3$, $OCF_3$, $SCF_3$, $SO_2$-$C_1$-$C_6$-Alkyl; das geradkettig oder verzweigt und durch Fluor ganz oder teilweise substituiert sein kann, $N(CF_3)_2$, einen Phenyl- oder Phenoxyrest mit $CF_3$ oder CN in 4-Position und gegebenenfalls weiteren Substituenten, 1,1,1,3,3,3-Hexafluorpropoxy, 1,1,2-Trifluor-2-chlor-ethoxy, 1,1,2,2-Tetrafluorethoxy, 1,1,2-Trifluor-2-chlor-ethylthio oder 1,1,2,3,3,3-Hexafluorpropylthio, unabhängig davon

$R^2$ für F, Cl, Br, CN, $CH_3$, $OCF_3$, $SO_2$-$C_1$-$C_6$-Alkyl, das geradkettig oder verzweigt und durch Fluor ganz oder teilweise substituiert sein kann, COO-$C_1$-$C_6$-Alkyl, $COOC_6H_5$, 1,1,2,2-Tetrafluorethoxy, 1,1,2,3,3,3-Hexafluorpropoxy oder 1,1,2-Trifluor-2-chlor-ethoxy und

$R^3$ für Wasserstoff, $COCH_3$ oder $COCF_3$ stehen, wobei weiterhin

$R^1$ und $R^2$ gemeinsam für einen -O-CFCl-CFCl-O-Rest stehen können,

mit Ausnahme von Verbindungen, bei denen

$R^1$ für 1,1,2,2-Tetrafluorethoxy und $R^2$ für Cl oder Brom oder

$R^1$ für $CF_3$ oder 1,1,2,2-Tetrafluorethoxy und $R^2$ für $CF_3$, $OCF_3$, 1,1,2,2-Trifluorethoxy oder 1,1,2-Trifluor-2-chlorethoxy stehen und wobei mindestens einer der Reste $R^1$, $R^2$ und $R^3$ im Rahmen die oben gegebenen Definitionen für eine Fluoralkyl(en) enthaltende Gruppe steht.

**2.** Fluoralkyl(en)gruppen enthaltende o-Phenylendiamine nach Anspruch 1, dadurch gekennzeichnet, daß

$R^3$ für Wasserstoff steht und die Reste $R^1$ und $R^2$ folgende Bedeutungen in der jeweils angegebenen Position haben

| $R^1$ | $R^2$ | $R^1$ | $R^2$ |
|---|---|---|---|
| 4-$CF_3O$ | 5-Br | H | 4-$N(CF_3)_2$ |
| 4-$CF_3O$ | 5-Cl | H | 4-$CF_3SO_2$ |
| 4-$CF_3S$ | 5-Cl | 4-$CF_3$ | 6-$COOCH_3$ |
| | | $CF_3$ | 6-$COOC_2H_5$ |
| 3-$CF_3$ | 5-$CH_3$ | 4-$CF_3$ | 6-$COOiC_3H_7$ |
| 3-$CF_3$ | 5-Cl | 4-$CF_3$ | 6-$COOC_6H_5$ |
| 3-$CF_3$ | 5-Br | 4-$CF_3$ | 5-$COOCH_3$ |
| 3-$CF_3$ | 5-CN | 4-$CF_3$ | 5-$COOC_2H_5$ |
| 5-$CF_3S$ | 3-Br | 4-$CF_3$ | 5-$COOtC._4H_9$ |
| 5-$CF_3$ | 3-CN | 5-$CF_3S$ | 3-Br |
| 3-$CF_3$ | 4-Cl | 5-$CF_3S$ | 3-Cl |
| 3-$CF_3O$ | 4-Cl | 5-$CF_3O$ | 3-$COOCH_3$ |
| 3-$CF_3$ | 4-CN | 5-$CF_3O$ | 3-$COOC_2H_5$ |
| 5-(2,6-dichlor-4-trifluormethyl-phenoxy) | 3-Cl | 5-$CF_3O$ | 3-$COOiC_3H_7$ |
| | | 5-$CFClHCF_2S$ | 3-$COOCH_3$ |
| 5-(2,6-dichlor-4-trifluormethyl-phenoxy) | 4-Cl | 5-$CFClHCF_2S$ | 3-$COOC_2H_5$ |
| | | 5-$CFClHCF_2S$ | 3-$COOiC_3H_7$ |
| | | 5-$CFClHCF_2S$ | 3-Br |
| | | 5-Hexafluorpropylthio | 3-Br |
| 4-Hexafluorpropoxy | 5-Cl | 3-$CF_3$ | 5-$COOC_4H_9$ |
| | | 5-$CF_3O$ | 3-$COOCH_3$ |
| 5-$CF_3O$ | 3-$COOC_2H_5$ | 5-$CF_3O$ | 3-$COOC_4H_9$ |
| | | 4-$CF_3CHFCF_2O$ | 5-$CF_3CHFCF_2O$ |
| 4-$CFClHCF_2O$ | 5-$CFClHCF_2O$ | 5-$CFClHCF_2O$ | 4-Cl |
| 4-$CF_3$ | 5-$CF_3$-CHF-CFO | | |
| | | 5-$CF_3SO_2$ | 3-Br |
| | | 5-$CF_3SO$ | 3-Cl |
| | | | |

**3.** Eine Fluoralkylengruppe enthaltende o-Phenylendiamine der Formel (I')

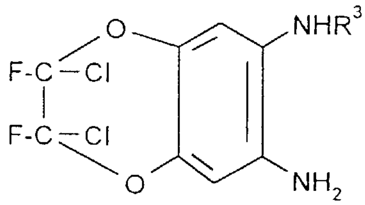

(I'),

in der

$R^3$ die in Anspruch 1 angegebene Bedeutung hat.

**4.** Verfahren zur Herstellung von Fluoralkyl(en)gruppen enthaltenden o-Phenylendiaminen des Anspruchs 1, bei denen $R^1$ und $R^2$ je eine Donorgruppe in 4- und 5-Stellung zu den Aminogruppen darstellen und $R^3$ für Wasserstoff steht, dadurch gekennzeichnet, daß man ein Benzolderivat der Formel (II)

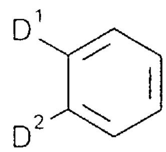

(II),

in der

$D^1$ für $CF_3O$, $CF_3S$, $CHF_2CF_2O$, $CHFCl\text{-}CF_2O$, $CF_3CHFCF_2O$, $CF_3CF_2O$, $CF_3CF_2CF_2O$, $CF_3CF_2S$ oder $CF_3CHFCF_2O$ und

$D^2$ für $CF_3O$, $CF_3S$, $CHF_2CF_2O$, $CHFCl\text{-}CF_2O$, $CF_3CHF\text{-}CF_2O$, $CF_3CF_2O$, $CF_3CF_2CF_2O$, $CF_3CF_2S$, $CF_3CHFCF_2O$, Fluor, Chlor, Brom, $C_1$-$C_6$-Alkyl oder $C_1$-$C_6$-Alkoxy steht,

dinitriert, die Nitrogruppen anschließend reduziert und so Verbindungen der Formel (I) erhält, bei denen $R^1$ und $R^2$ in 4- und 5-Stellung zu den Aminogruppen stehen und die oben angegebene Bedeutung von $D^1$ und $D^2$ haben.

**5.** Verfahren nach Anspruch 4, dadurch gekennzeichnet, daß man die Dinitrierung mit $HNO_3/H_2SO_4$-Gemischen, die gegebenenfalls Oleum enthalten, bei Temperaturen von 0 bis 100°C und dieReduktion mit Eisen in Gegenwart von wäßriger Salzsäure und Ethanol bei 50 bis 100°C oder katalytisch mit elementarem Wasserstoff bei 25 bis 100°C, 1 bis 50 bar und in Gegenwart von Metallen oder Metallverbindungen der 8. Nebengruppe des periodischen Systems durchführt.

**6.** Verfahren zur Herstellung von Fluoralkyl(en)gruppen enthaltenden o-Phenylendiaminen des Anspruchs 1, bei denen $R^1$ die in Anspruch 1 angegebene Bedeutung hat und in 4-Stellung zu den Aminogruppen steht, $R^2$ für Cl oder Br in 5-Stellung zu den Aminogruppen steht und $R^3$ Wasserstoff bedeutet, dadurch gekennzeichnet, daß man ein Nitrobenzolderivat der Formel (III) in der

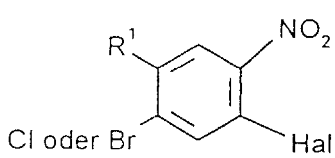

(III),

$R^1$ die bei Formel (I) angegebene Bedeutung hat und Hal für Fluor, Chlor oder Brom steht,

mit Ammoniak umsetzt, so die Hal-Gruppe gegen eine Aminogruppe austauscht und das so erhaltene Nitranilin

reduziert.

**7.** Verfahren nach Anspruch 6, dadurch gekennzeichnet, daß man die Umsetzung mit Ammoniak mit flüssigen Ammoniak in Gegenwart von Wasser und einen Tetraalkylammoniumsalz bei Temperaturen von 80 bis 200°C in einem Druckgefäß und die Reduktion analog zu Anspruch 5 durchgeführt.

**8.** Verfahren zur Herstellung von Fluoralkyl(en)gruppen enthaltenden o-Phenylendiaminen des Anspruchs 1, bei denen R1 die in Anspruch 1 angegebene Bedeutung hat, $R^2$ für Cl oder Br in 6-Stellung zu den Aminogruppen steht und $R^3$ Wasserstoff bedeutet, dadurch gekennzeichnet, daß man ein Nitranilin der Formel (IV)

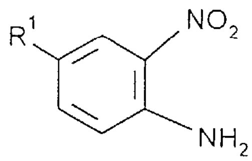

(IV),

in der

$R^1$ die in Anspruch 1 angegebene Bedeutung hat,

mit einem Chlorierungs- oder Bromierungsmittel umsetzt und anschließend die Nitrogruppe reduziert.

**9.** Verfahren zur Herstellung von Fluoralkyl(en)gruppen enthaltenden o-Phenylendiaminen des Anspruch 1, bei denen $R^1$ eine Donorgruppe in 4-Stellung zu den beiden Aminogruppen und $R^2$ eine Akzeptorgruppe darstellt und $R^3$ verschieden von Wasserstoff ist, dadurch gekennzeichnet, daß man ein Benzolderivat der Formel V

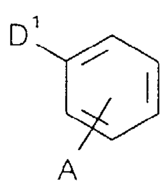

(V),

in der

$D^1$ die in Anspruch 4 angegebene Bedeutung hat und

A für $CF_3$, $SO_2$-$C_1$-$C_6$-Alkyl, das geradkettig oder verzweigt und durch Fluor ganz oder teilweise substituiert sein kann, COO-$C_1$-$C_6$-Alkyl oder CN

steht,
mononitriert, die $NO_2$-Gruppe zur $NH_2$-Gruppe reduziert, die $NH_2$-Gruppe acyliert, nochmals mononitriert und auch diese $NO_2$-Gruppe zur $NH_2$-Gruppe reduziert.

**10.** Verwendung von Fluoralkyl(en)gruppen enthaltenden o-Phenylendiaminen des Anspruchs 1, in denen $R^3$ Wasserstoff bedeutet oder durch Verseifung in Wasserstoff umgewandelt wird, zur Herstellung von substituierten Benzimidazolen der Formel

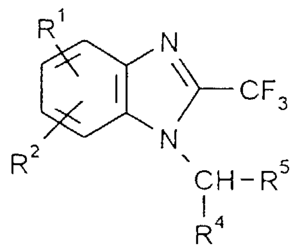

(VI)

indem man Stoffe der Formel (I) zunächst mit Trifluoressigsäure zu 2-Trifluormethyl-benzimidazolen der Formel

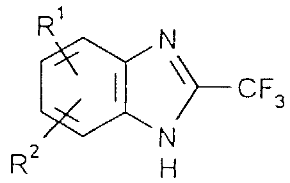

(VII)

umsetzt und dann eine weitere Umsetzung mit Verbindungen der Formel

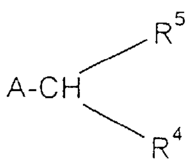

(VIII)

vornimmt, wobei in den Formeln (VI), (VII) und (VIII) $R^1$ und $R^2$ den in Anspruch 1 genannten Bedeutungsumfang annehmen,

$R^4$ für Wasserstoff, Alkyl, Alkoxy oder für gegebenenfalls substituiertes Aryl steht,

$R^5$ für Hydroxy, Cyano oder für jeweils gegebenenfalls substituiertes Alkyl, Alkenyl, Alkinyl, Alkoxy, Alkenyloxy, Alkinyloxy, Alkylthio, Amino, Aminocarbonyl, Alkylcarbonyl, Alkoxycarbonyl, Alkylcarbonyloxy, Dialkoxyphosphonyl,(Hetero)Aryl, (Hetero)-Arylcarbonyl, (Hetero)Aryl oxycarbonyl, (Hetero)Arylcarbonyloxy oder (Hetero) Aryl aminocarbonyl aminocarbonyl oxy steht und

A eine geeignete Abggangsgruppe bedeutet.

## Claims

**1.** o-Phenylenediamines of the formula (I) containing fluoroalkyl(ene) groups

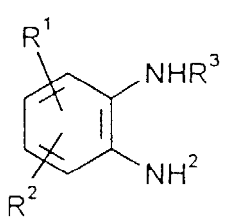

(I)

in which

$R^1$ represents $CF_3$, $OCF_3$, $SCF_3$, $SO_2$-$C_1$-$C_6$-alkyl, which can be straight-chain or branched and be substituted

completely or in part by fluorine, $N(CF_3)_2$, a phenyl or phenoxy radical containing $CF_3$ or CN in the 4 position and, if desired, further substituents, 1,1,2,3,3,3-hexafluoropropoxy, 1,1,2-trifluoro-2-chloro-ethoxy, 1,1,2,2-tetrafluoroethoxy, 1,1,2-trifluoro-2-chloro-ethylthio or 1,1,2,3,3,3-hexafluoropropylthio, independently thereof

$R^2$ represents F, Cl, Br, CN, $CH_3$, $OCF_3$, $SO_2$-$C_1$-$C_6$-alkyl, which can be straight-chain or branched and substituted completely or in part by fluorine, COO-$C_1$-$C_6$-alkyl, $COOC_6H_5$, 1,1,2,2-tetrafluoroethoxy, 1,1,2,3,3,3-hexafluoropropoxy or 1,1,2-trifluoro-2-chloro-ethoxy, and

$R^3$ represents hydrogen, $COCH_3$ or $COCF_3$, it furthermore being possible for

$R^1$ and $R^2$together to represent an -O-CFCl-CFCl-O-radical,

with the exception of compounds in which

$R^1$ represents 1,1,2,2-tetrafluoroethoxy and $R^2$ represents C1 or bromine or

$R^1$ represents $CF_3$ or 1,1,2,2-tetrafluoroethoxy and $R^2$ represents $CF_3$, $OCF_3$, 1,1,2,2-trifluoroethoxy or 1,1,2-trifluoro-2-chloroethoxy and at least one of the radicals $R^1$, $R^2$ and $R^3$ represents a fluoroalkyl(ene)-containing group within the scope of the definitions given above.

**2.** o-Phenylenediamines according to Claim 1 containing fluoroalkyl(ene) groups, characterized in that

$R^3$ represents hydrogen, and the radicals $R^1$ and $R^2$ have the following meanings in the position given in each case

| $R^1$ | $R^2$ | $R^1$ | $R^2$ |
|---|---|---|---|
| 4-$CF_3O$ | 5-Br | H | 4-$N(CF_3)_2$ |
| 4-$CF_3O$ | 5-Cl | H | 4-$CF_3SO_2$ |
| 4-$CF_3S$ | 5-Cl | 4-$CF_3$ | 6-$COOCH_3$ |
| | | $CF_3$ | 6-$COOC_2H_5$ |
| 3-$CF_3$ | 5-$CH_3$ | 4-$CF_3$ | 6-$COOiC_3H_7$ |
| 3-$CF_3$ | 5-Cl | 4-$CF_3$ | 6-$COOC_6H_5$ |
| 3-$CF_3$ | 5-Br | 4-$CF_3$ | 5-$COOCH_3$ |
| 3-$CF_3$ | 5-CN | 4-$CF_3$ | 5-$COOC_2H_5$ |
| 5-$CF_3S$ | 3-Br | 4-$CF_3$ | 5-COOt.$C_4H_9$ |
| 5-$CF_3$ | 3-CN | 5-$CF_3S$ | 3-Br |
| 3-$CF_3$ | 4-Cl | 5-$CF_3S$ | 3-Cl |
| 3-$CF_3O$ | 4-Cl | 5-$CF_3O$ | 3-$COOCH_3$ |
| 3-$CF_3$ | 4-CN | 5-$CF_3O$ | 3-$COOC_2H_5$ |
| 5-(2,6-dichloro-4-trifluoromethylphenoxy) | 3-Cl | 5-$CF_3O$ | 3-$COOiC_3H_7$ |
| | | 5-CFC1H$CF_2S$ | 3-$COOCH_3$ |
| 5-(2,6-dichloro-4-trifluoromethyl-phenoxy) | 4-Cl | 5-CFC1H$CF_2S$ | 3-$COOC_2H_5$ |
| | | 5-CFC1H$CF_2S$ | 3-$COOiC_3H_7$ |
| | | 5-CFC1H$CF_2S$ | 3-Br |
| | | 5-Hexafluorpropylthio | 3-Br |
| 4-Hexafluoropropoxy | 5-Cl | 3-$CF_3$ | 5-$COOC_4H_9$ |

(continued)

| $R^1$ | $R^2$ | $R^1$ | $R^2$ |
|---|---|---|---|
| | | 5-$CF_3O$ | 3-$COOCH_3$ |
| 5-$CF_3O$ | 3-$COOC_2H_5$ | 5-$CF_3O$ | 3-$COOC_4H_9$ |
| | | 4-$CF_3CHFCF_2O$ | 5-$CF_3CHFCF_2O$ |
| 4-$CFClHCF_2O$ | 5-$CFClHCF_2O$ | 5-$CFC1HCF_2O$ | 4-Cl |
| 4-$CF_3$ | 5-$CF_3$-CHF-CFO | | |
| | | 5-$CF_3SO_2$ | 3-Br |
| | | 5-$CF_3SO$ | 3-Cl |
| | | | |

**3.** o-Phenylenediamines of the formula (I') containing a fluoroalkylene group

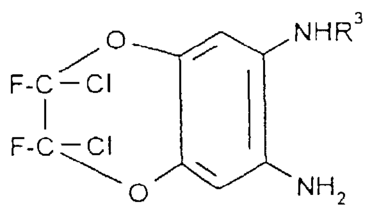

(I'),

in which

$R^3$ has the meaning given in Claim 1.

**4.** Process for preparing o-phenylenediamines of Claim 1 containing fluoroalkyl(ene) groups, in which $R^1$ and $R^2$ each represent a donor group in the 4 and 5 position relative to the amino groups and $R^3$ represents hydrogen, characterized in that a benzene derivative of the formula (II)

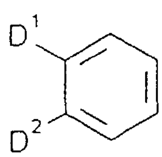

(II),

in which

$D^1$ represents $CF_3O$, $CF_3S$, $CHF_2CF_2O$, $CHFCl-CF_2O$, $CF_3CHFCF_2O$, $CF_3CF_2O$, $CF_3CF_2CF_2O$, $CF_3CF_2S$ or $CF_3CHFCF_2O$ and

$D^2$ represents $CF_3O$, $CF_3S$, $CHF_2CF_2O$, $CHFCl-CF_2O$, $CF_3CHF-CF_2O$, $CF_3CF_2O$, $CFCF_2CF_2O$, $CF_3CF_2S$, $CF_3CHFCF_2O$, fluorine, chlorine, bromine, $C_1$-$C_6$-alkyl or $C_1$-$C_6$-alkoxy,

is dinitrated, the nitro groups are then reduced, thus obtaining compounds of the formula (I) in which $R^1$ and $R^2$ are in the 4 and 5 position relative to the amino groups and have the abovementioned meaning of $D^1$ and $D^2$.

**5.** Process according to Claim 4, characterized in that dinitration is effected with $HNO_3/H_2SO_4$ mixtures, which, if desired, contain oleum, at temperatures of 0 to 100°C, and the reduction is effected with iron in the presence of aqueous hydrochloric acid and ethanol at 50 to 100°C or catalytically with elemental hydrogen at 25 to 100°C, 1 to 50 bar and in the presence of metals or metal compounds from subgroup VIII of the Periodic Table.

6. Process for preparing o-phenylenediamines of Claim 1 containing fluoroalkyl(ene) groups, in which $R^1$ has the meaning given in Claim 1 and is in the 4 position relative to the amino groups, $R^2$ represents Cl or Br in the 5 position relative to the amino groups, and $R^3$ denotes hydrogen, characterized in that a nitrobenzene derivative of the formula (III)

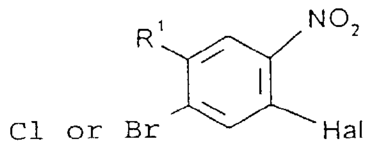

(III),

in which

$R^1$ has the meaning given in formula (I) and Hal represents fluorine, chlorine or bromine,

is reacted with ammonia, thus exchanging the Hal group for an amino group, and the nitroaniline thus obtained is reduced.

7. Process according to Claim 6, characterized in that the reaction with ammonia is carried out with liquid ammonia in the presence of water and a tetraalkylammonium salt at temperatures of 80 to 200°C in a pressure vessel, and the reduction is carried out analogously to Claim 5.

8. Process for preparing o-phenylenediamines of Claim 1 containing fluoroalkyl(ene) groups, in which $R^1$ has the meaning given in Claim 1, $R^2$ represents Cl or Br in the 6 position relative to the amino groups, and $R^3$ denotes hydrogen, characterized in that a nitroaniline of the formula (IV)

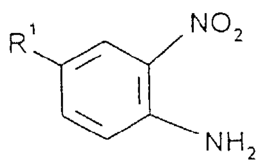

(IV),

in which

$R^1$ has the meaning given in Claim 1,

is reacted with a chlorinating or brominating agent, followed by reduction of the nitro groups.

9. Process for preparing o-phenylenediamines of Claim 1 containing fluoroalkyl(ene) groups, in which $R^1$ is a donor group in the 4 position relative to the two amino groups, and $R^2$ represents an acceptor group, and $R^3$ is different from hydrogen, characterized in that a benzene derivative of the formula V

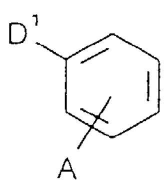

(V),

in which

$D^1$ has the meaning given in Claim 4, and

A represents $CF_3$, $SO_2$-$C_1$-$C_6$-alkyl, which can be straight-chain or branched and be substituted completely or

in part by fluorine, COO-$C_1$-$C_6$-alkyl or CN,

is mononitrated, the $NO_2$ group is reduced to the $NH_2$ group, the $NH_2$ group is acylated, the resulting compound is again mononitrated, and this $NO_2$ group is also reduced to the $NH_2$ group.

**10.** Use of o-phenylenediamines of Claim 1 containing fluoroalkyl(ene) groups, in which $R^3$ denotes hydrogen or is converted into hydrogen by hydrolysis, for preparing substituted benzimidazoles of the formula

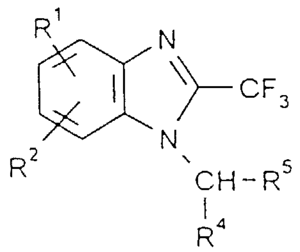

(VI)

by reacting substances of the formula (I) first with trifluoroacetic acid to give 2-trifluoromethylbenzimidazoles of the formula

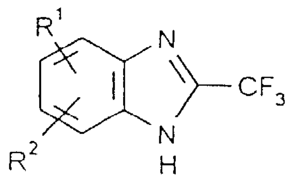

(VII)

which are then further reacted with compounds of the formula

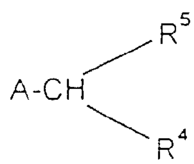

(VIII)

in which in formulae (VI), (VII) and (VIII) $R^1$ and $R^2$ adopt the range of meanings mentioned in Claim 1,

$R^4$ represents hydrogen, alkyl, alkoxy or substituted or unsubstituted aryl,

$R^5$ represents hydroxyl, cyano or alkyl, alkenyl, alkinyl, alkoxy, alkenyloxy, alkinyloxy, alkylthio, amino, aminocarbonyl, alkylcarbonyl, alkoxycarbonyl, alkylcarbonyloxy, dialkoxyphosphonyl, (hetero) aryl, (hetero) arylcarbonyl, (hetero)aryloxycarbonyl, (hetero)arylcarbonyloxy or (hetero)arylaminocarbonylaminocarbonyloxy, each of which is substituted or unsubstituted, and

A denotes a suitable leaving group.

## Revendications

**1.** o-phénylènediamines portant des groupes fluoralkyl(ène), de formule (I)

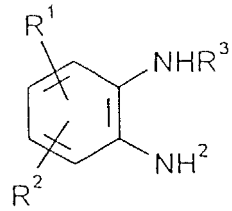

(I)

dans laquelle

$R^1$ représente $CF_3$, $OCF_3$, $SCF_3$, $SO_2$-(alkyle en $C_1$ à $C_6$), qui peut être linéaire ou ramifié et substitué entièrement ou partiellement par du fluor, $N(CF_3)_2$, un reste phényle ou phénoxy avec un groupe $CF_3$ ou CN en position 4 et le cas échéant d'autres substituants, un groupe 1,1,2,3,3,3hexafluoropropoxy, 1,1,2-trifluoro-2-chloréthoxy, 1,1,2,2-tétrafluoréthoxy, 1,1,2-trifluoro-2-chloréthylthio ou 1,1,2,3,3,3-hexafluoropropylthio, indépendamment de quoi

$R^2$ représente F, Cl, Br, un groupe CN, $CH_3$, $OCF_3$, $SO_2$-(alkyle en $C_1$ à $C_6$) qui est linéaire ou ramifié et peut être substitué en totalité ou en partie par du fluor, un groupe COO-(alkyle en $C_1$ à $C_6$), $COOC_6H_5$, 1,1,2,2-tétrafluoréthoxy, 1,1,2,3,3,3-hexafluoropropoxy ou 1,1,2-trifluoro-2-chloréthoxy et

$R^3$ est de l'hydrogène, un groupe $COCH_3$ ou $COCF_3$, en outre

$R^1$ et $R^2$ peuvent former conjointement un reste -O-CFCl-CFCl-O-,

et à l'exception de composés dans lesquels

$R^1$ est un groupe 1,1,2,2-tétrafluoréthoxy et $R^2$ représente Cl ou du brome, ou bien

$R^1$ est un groupe $CF^3$ ou un groupe 1,1,2,2-tétrafluoréthoxy et $R^2$ est un groupe $CF_3$, $OCF_3$, 1,1,2,2-trifluoréthoxy ou 1,1,2-trifluoro-2-chloréthoxy et l'un au moins des restes $R^1$, $R^2$ et $R^3$ représente dans le cadre des définitions indiquées ci-dessus un groupe contenant un radical fluoralkyl(ène).

**2.** o-phénylènediamines contenant des groupes fluoralkyl(ène) suivant la revendication 1, caractérisées en ce que

$R^3$ représente de l'hydrogène et les restes $R^1$ et $R^2$ ont les définitions suivantes dans la position indiquée dans chaque cas,

| $R^1$ | $R^2$ | $R^1$ | $R^2$ |
|---|---|---|---|
| 4-$CF_3O$ | 5-Br | H | 4-$N(CF_3)_2$ |
| 4-$CF_3O$ | 5-Cl | H | 4-$CF_3SO_2$ |
| 4-$CF_3S$ | 5-Cl | 4-$CF_3$ | 6-$COOCH_3$ |
| | | $CF_3$ | 6-$COOC_2H_5$ |
| 3-$CF_3$ | 5-$CH_3$ | 4-$CF_3$ | 6-$COOiC_3H_7$ |
| 3-$CF_3$ | 5-Cl | 4-$CF_3$ | 6-$COOC_6H_5$ |
| 3-$CF_3$ | 5-Br | 4-$CF_3$ | 5-$COOCH_3$ |
| 3-$CF_3$ | 5-CN | 4-$CF_3$ | 5-$COOC_2H_5$ |
| 5-$CF_3S$ | 3-Br | 4-$CF_3$ | 5-COOt.$C_4H_9$ |
| 5-$CF_3$ | 3-CN | 5-$CF_3S$ | 3-Br |
| 3-$CF_3$ | 4-Cl | 5-$CF_3S$ | 3-Cl |
| 3-$CF_3O$ | 4-Cl | 5-$CF_3O$ | 3-$COOCH_3$ |
| 3-$CF_3$ | 4-CN | 5-$CF_3O$ | 3-$COOC_2H_5$ |
| 5-(2,6-dichloro-4-trifluorométhyl-phénoxy) | 3-Cl | 5-$CF_3O$ | 3-$COOiC_3H_7$ |

(suite)

| $R^1$ | $R^2$ | $R^1$ | $R^2$ |
|---|---|---|---|
| | | 5-$CFClHCF_2S$ | 3-$COOCH_3$ |
| 5-(2,6-dichloro-4-trifluorométhyl-phénoxy) | 4-Cl | 5-$CFClHCF_2S$ | 3-$COOC_2H_5$ |
| | | 5-$CFClHCF_2S$ | 3-$COOiC_3H_7$ |
| | | 5-$CFClHCF_2S$ | 3-Br |
| | | 5-Hexafluorpropylthio | 3-Br |
| 4-Hexafluoropropoxy | 5-Cl | 3-$CF_3$ | 5-$COOC_4H_9$ |
| | | 5-$CF_3O$ | 3-$COOCH_3$ |
| 5-$CF_3O$ | 3-$COOC_2H_5$ | 5-$CF_3O$ | 3-$COOC_4H_9$ |
| | | 4-$CF_3CHFCF_2O$ | 5-$CF_3CHFCF_2O$ |
| 4-$CFClHCF_2O$ | 5-$CFClHCF_2O$ | 5-$CFClHCF_2O$ | 4-Cl |
| 4-$CF_3$ | 5-$CF_3$-CHF-CFO | | |
| | | 5-$CF_3SO_2$ | 3-Br |
| | | 5-$CF_3SO$ | 3-Cl |
| | | | |

**3.** o-phénylènediamines contenant un groupe fluoralkylène de formule (I')

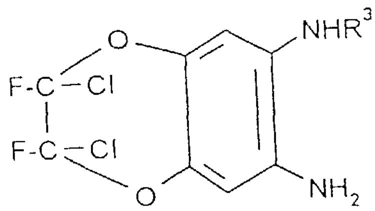

(I'),

dans laquelle

$R^3$ a la définition indiquée dans la revendication 1.

**4.** Procédé de production de o-phénylènediamines contenant des groupes fluoralkyl(ène) suivant la revendication 1, dans lesquelles $R^1$ et $R^2$ représentent chacun un groupe donneur en position 4 et 5 par rapport aux groupes amino et $R^3$ représente de l'hydrogène, caractérisé en ce qu'on dinitre un dérivé benzénique de formule (II)

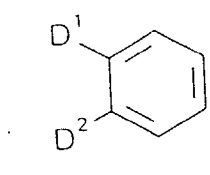

(II),

dans laquelle

$D^1$ est un groupe $CF_3O$, $CF_3S$, $CHF_2CF_2O$, $CHFCl$-$CF_2O$, $CF_3CHFCF_2O$, $CF_3CF_2O$, $CF_3CF_2CF_2O$, $CF_3CF_2S$ ou $CF_3CHFCF_2O$ et

$D^2$ représente un groupe $CF_3O$, $CF_3S$, $CHF_2CF_2O$, $CHFCl$-$CF_2O$, $CF_3CHF$-$CF_2O$, $CF_3CF_2O$, $CFCF_2CF_2O$, $CF_3CF_2S$, $CF_3CHFCF_2O$, du fluor, du chlore, du brome, un groupe alkyle en $C_1$ à $C_6$ ou un groupe alkoxy en $C_1$ à $C_6$,

on réduit ensuite les groupes nitro et on obtient ainsi des composés de formule (I) dans lesquels $R^1$ et $R^2$ sont en position 4 et 5 par rapport aux groupes amino et ont la définition indiquée ci-dessus pour $D^1$ et $D^2$.

**5.** Procédé suivant la revendication 4, caractérisé en ce qu'on conduit la dinitration avec des mélanges $HNO_3/H_2SO_4$ qui contiennent éventuellement un oléum, à des températures de 0 à 100°C et on conduit la réduction avec du fer en présence d'acide chlorhydrique aqueux et d'éthanol à 50-100°C ou par voie catalytique avec de l'hydrogène élémentaire à 25-100°C sous pression de 1 à 50 bars et en présence de métaux ou de composés métalliques du huitième sous-groupe du système périodique.

**6.** Procédé de production de o-phénylènediamines contenant des groupes fluoralkyl(ène) suivant la revendication 1, dans lesquelles $R^1$ a la définition indiquée dans la revendication 1 et est présent en position 4 par rapport aux groupes amino, $R^2$ est du chlore ou du brome en position 5 par rapport aux groupes amino et $R^3$ représente de l'hydrogène, caractérisé en ce qu'on fait réagir avec l'ammoniac un dérivé de nitrobenzène de formule (III)

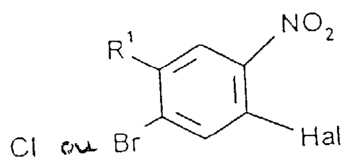

(III),

dans laquelle

$R^1$ a la définition indiquée pour la formule (I) et Hal représente du fluor, du chlore ou du brome,

on échange ainsi le groupe Hal contre un groupe amino et on réduit la nitraniline ainsi obtenue.

**7.** Procédé suivant la revendication 6, caractérisé en ce qu'on conduit la réaction avec l'ammoniac avec de l'ammoniac liquide en présence d'eau et d'un sel tétra-alkylammonium à des températures de 80 à 200°C dans un récipient sous pression et on conduit la réduction par analogie avec la revendication 5.

**8.** Procédé de production de o-phénylènediamines contenant des groupes fluoralkyl(ène) suivant la revendication 1, dans lesquelles $R^1$ a la définition indiquée dans la revendication 1, $R^2$ représente Cl ou Br en position 6 par rapport aux groupes amino et $R^3$ désigne de l'hydrogène, caractérisé en ce qu'on fait réagir une nitraniline de formule (IV)

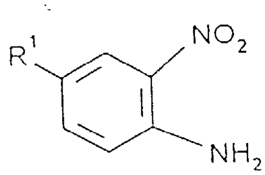

(IV),

dans laquelle

$R^1$ a la définition indiquée dans la revendication 1,

avec un agent de chloration ou de bromation puis on réduit le groupe nitro.

**9.** Procédé de production de o-phénylènediamines contenant des groupes fluoralkyl(ène) suivant la revendication 1, dans lesquelles $R^1$ est un groupe donneur en position 4 par rapport aux deux groupes amino et $R^2$ est un groupe accepteur et $R^3$ ne représente pas de l'hydrogène, caractérisé en ce qu'on mononitre un dérivé de benzène de formule V

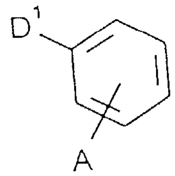

(V),

dans laquelle

$D^1$ a la définition indiquée dans la revendication 4 et
A est un groupe $CF_3$, un groupe $SO_2$-(alkyle en $C_1$ à $C_6$) qui est linéaire ou ramifié et peut être substitué en totalité ou en partie par du fluor, un groupe COO-(alkyle en $C_1$ à $C_6$) ou un groupe CN,

on réduit le groupe $NO_2$ en groupe $NH_2$, on acyle le groupe $NH_2$, on effectue une nouvelle mononitration et on réduit aussi ce groupe $NO_2$ en groupe $NH_2$.

**10.** Utilisation de o-phénylènediamines contenant des groupes fluoralkyl(ène) suivant la revendication 1, dans lesquelles $R^3$ représente de l'hydrogène ou est transformé en hydrogène par saponification, pour la production de benzimidazoles substitués de formule

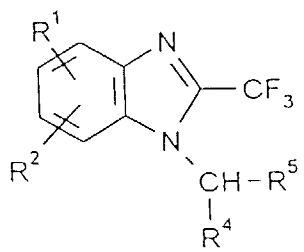

(VI)

en faisant réagir des composés de formule (I) tout d'abord avec l'acide trifluoracétique pour former des 2-trifluorométhylbenzimidazoles de formule

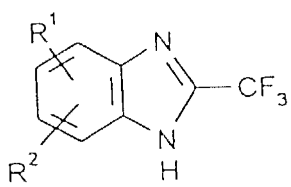

(VII)

puis en poursuivant la réaction avec des composés de formule

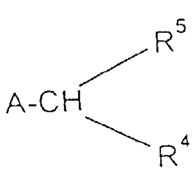

(VIII)

formules (VI), (VII) et (VIII) dans lesquelles $R^1$ et $R^2$ ont le cadre de définition mentionné dans la revendication 1,

$R^4$ est de l'hydrogène, un groupe alkyle, alkoxy ou un groupe aryle facultativement substitué,
$R^5$ est un groupe hydroxy, un groupe cyano ou un groupe alkyle, alcényle, alcynyle, alkoxy, alcényloxy, alcynyloxy, alkylthio, amino, aminocarbonyle, alkylcarbonyle, alkoxycarbonyle, alkylcarbonyloxy, dialkoxyphosphonyle, (hétéro)aryle, (hétéro)arylcarbonyle, (hétéro)aryloxycarbonyle, (hétéro)arylcarbonyloxy ou (hétéro) arylaminocarbonylaminocarbonyloxy dont chacun est éventuellement substitué, et

A est un groupe partant approprié.